# EUROPÄISCHE PATENTSCHRIFT

(11) **EP 0 571 851 B1**
(45) Veröffentlichungstag und Bekanntmachung des Hinweises auf die Patenterteilung: **17.04.1996**
(21) Anmeldenummer: 93107973.5
(22) Anmeldetag: 17.05.1993
(51) Int. Cl.: C07D 233/32, C07D 239/10, C07D 243/04, C07D 245/02, C07C 67/03

(54) **Verfahren zur Herstellung von endständig Stickstoffheterocyclus-substituiertem (Meth)acrylsäureester**
Process for the preparation of terminal nitrogen-heterocyclic substituted acrylic- and methacrylicacid alkylesters
Procédé de préparation d'esters acryliques et méthacryliques à terminaison nitrogen-hétérocycliques substitués

(30) Priorität: 23.05.1992 DE 4217124
(43) Veröffentlichungstag der Anmeldung: 01.12.1993
(73) Patentinhaber: RÖHM GMBH, D-64293 Darmstadt (DE)
(72) Erfinder: Knebel, Joachim, Dr., W-6100 Darmstadt (DE); Arndt, Peter Joseph, Dr., W-6104 Seeheim-Jugenheim (DE); Ude, Werner, Dr., W-6100 Darmstadt-Arheilgen (DE)

(56) Entgegenhaltungen:
- EP-A- 0 236 994
- EP-A- 0 433 135
- EP-A- 0 453 638
- US-A- 2 871 223
- US-A- 4 672 105
- US-A- 4 745 213

## Beschreibung

### Gebiet der Erfindung

Die vorliegende Erfindung betrifft ein neues und verbessertes Verfahren zur Herstellung von Acryl- oder Methacrylsäureestern der Formel 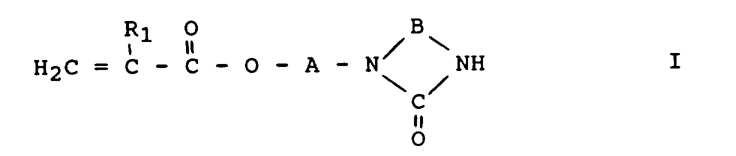 in der R₁ für Wasserstoff oder eine Methylgruppe steht und A und B unverzweigte oder verzweigte Alkylengruppen mit 2 bis 5 C-Atomen bedeuten.

### Stand der Technik

Verbindungen der Formel I lassen sich nach dem in der US-Patentschrift 2 871 223 beschriebenen Verfahren durch Umsetzung von Acryl- oder Methacrylsäurechlorid mit Hydroxialkylimidazolidin-2-onen in Gegenwart tertiärer Stickstoffbasen, wobei stöchiometrische Mengen der Hydrochloride der tertiären Stickstoffbasen mit anfallen, erhalten.
Bei dem aus der EP 0 236 994 A1 bekannten Verfahren zur Herstellung von Acryl- und Methacrylestern der Formel I werden Acryl- oder Methacrylsäureester mit 1-(Hydroxialkyl)-imidazolidin-2-onen in Gegenwart von Titanalkoholaten oder Chelatverbindungen der Metalle Titan, Zirkonium, Eisen und Zink mit 1,3-Dicarbonylverbindungen als Umesterungskatalysatoren umgesetzt.

In den EP-A 0 433 135 und EP-A 0 453 638 werden für die Umesterung von Acryl- und Methacrylestern mit Hydroxialkylimidazolidin-2-onen zu Verbindungen der Formel I, Diorganozinnoxi-Verbindungen als Umesterungskatalysatoren beansprucht.

In der Regel muß aus den Ansätzen nach Beendigung der Umsetzung der Metallkatalysator abgetrennt werden. Dies geschieht vorteilhaft, so z.B. bei Verwendung von Tetraalkyltitanaten oder von Dialkylzinnoxiden durch Zusetzen von Wasser. Aus den Titanaten entstehen dabei Metall(hydr)oxide, wie z.B. TiO₂, die beispielsweise durch Filtrieren oder Zentrifugieren abgetrennt werden. Diese hydrolysierten Umesterungskatalysatoren können nach Abtrennung nicht mehr als solche erneut eingesetzt werden.
Die Dialkylzinnoxide lassen sich durch den Wasserzusatz zwar als solche wieder abtrennen und als Umesterungskatalysatoren erneut einsetzen. Jedoch muß dazu eine relativ große Wassermenge zunächst eingebracht werden, die dann wieder aus dem Reaktionsprodukt entfernt werden muß.

### Aufgabe und Lösung

Der Erfindung lag die Aufgabe zugrunde, ein katalytisches Verfahren zur Herstellung von Acryl- oder Methacrylsäureestern der Formel I durch Alkoholyse von (Meth)acrylsäurealkylestern mit Hydroxialkylimidazolidin-2-onen zu finden, bei denen der verwendete Katalysator ohne die Zugabe von Wasser aus dem Reaktionsgemisch abgetrennt und gegebenenfalls als solcher wieder eingesetzt werden kann.

Es wurde gefunden, daß die Umsetzung überraschenderweise gut mit Alkali-/Erdalkali-Verbindungen, die im wesentlichen als Oxide, Hydroxide, Carbonate und/oder als Salze von Carbonsäuren eingesetzt werden, und die als solche schon als Veresterungs- und Umesterungskatalysatoren bei entsprechenden Herstellungen von Alkyl(meth)acrylaten bekannt sind, durchgeführt werden kann, und daß die als Katalysatoren vorhandenen Alkali/Erdalkali-Verbindungen ohne Zusetzen von Wasser abtrennbar sind. Erfindungsgemäß hat sich als besonders vorteilhaft ein Katalysatorsystem KS bestehend aus der Kombination der Verbindungen A + B erwiesen,
wobei
- A: die Verbindungen LiₙY bedeutet, worin Y für Halogenid oder Chlorat oder für das Carbonat oder das Salz einer Carbonsäure mit 1 bis 6 Kohlenstoffatomen oder für ein Alkoxid mit 1 bis 4 Kohlenstoffatomen, Hydroxid oder für Sauerstoff steht, und n entsprechend der Wertigkeit von Y für 1 oder 2 steht,
- B: die Verbindung CaX_{q} bedeutet, worin X für Sauerstoff oder Chlorid und q entsprechend der Wertigkeit von X für 1 oder 2 steht,
mit der Maßgabe, daß mindestens einer der beiden anionischen Bestandteile X und Y Sauerstoff enthalten soll.

Die Erfindung betrifft ein Verfahren zur Herstellung von (Meth)Acrylester der Formel I 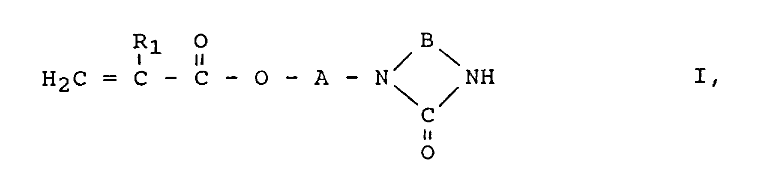 in der R₁ für Wasserstoff oder eine Methylgruppe steht und A und B unverzweigte oder verzweigte Alkylengruppen mit 2 bis 5 C-Atomen bedeuten, durch Umsetzung eines Acrylsäureesters oder Methacrylsäureesters der Formel II 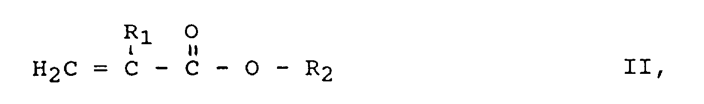 in der R₁ die oben angegebene Bedeutung und R₂ die Bedeutung eines Alkylrestes mit 1 bis 4 C-Atomen haben, mit einer heterocyclischen Verbindung der Formel III 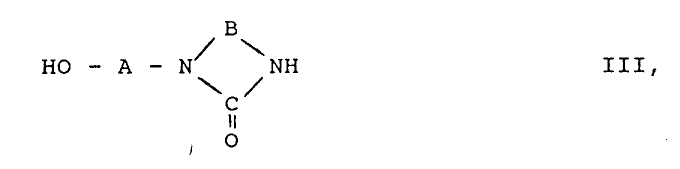 das dadurch gekennzeichnet ist, daß die Umsetzung eines Esters entsprechend der Formel II mit einer heterocyclischen Verbindung der Formel III zu einem Acryl- oder Methacrylester der Formel I in Gegenwart von 0,05 bis 1 Gew.-% (bezogen auf die Summe von II und III) eines Katalysatorsystems KS aus Lithium- und/oder Calcium-Verbindungen durchgeführt wird.

Die Herstellung wird insbesondere vorteilhaft mit einem Katalysatorsystem KS aus der Kombination der Verbindungen A + B durchgeführt, wobei A und B die oben angegebenen Bedeutungen haben. In der DE-OS 34 23 441 und in der DE-OS 34 23 443 werden Verfahren zur Herstellung von Estern beschrieben, wobei zur Durchführung verschiedener Esterherstellungs-Reaktionen Katalysatorsysteme KS verwendet werden, die aus solchen Lithium-/Calcium-Verbindungen aufgebaut sind.

Ein besonderer Vorteil des neuen Verfahrens ist, daß ohne Zugabe von Wasser oder anderer Abtrennhilfsmittel der Katalysator, das heißt Alkali- und/oder Erdalkali-Verbindungen, und der praktisch quantitativ in dem Reaktionsgemisch aufgeschlämmt ist, z.B. durch Filtration abgetrennt wird und dann wieder erneut als Katalysator eingesetzt werden kann.

Verbindungen der Formel I sind wertvolle Comonomere und werden beispielsweise bei der Herstellung von Polymerdispersionen aus Vinylmonomeren eingesetzt, die vor allem als Bindemittel z.B. in Lacken, oder als Lederhilfsmittel Verwendung finden. Comonomere der Formel I verleihen Copolymerisaten eine gewünschte Hydrophilie und können in wärmehärtbaren Harzen mit ihrer Imidgruppe als Formaldehydfänger fungieren.

Der Erfolg des erfindungsgemäßen Verfahrens ist überraschend, da aufgrund der Bifunktionalitäten von I und II bei deren Umsetzung mit weiteren Reaktionen, wie Additionsreaktionen analog einer Michaeladdition an die Doppelbindung, oder mit einer Amidbildung durch Reaktion des Acryl- oder Methacrylesters I mit der NH-Gruppierung einer Verbindung der Formel II in Gegenwart der relativ stark basischen Katalysatorkomponenten zu rechnen war. Die erfindungsgemäße Umsetzung von Acryl- und Methacrylestern der Formel II mit den Alkoholen der Formel III verläuft sehr selektiv zu Verbindungen der Formel I. Die Reaktionszeit ist gegenüber dem Verfahren mit Dibutylzinnoxid als Katalysator um ca. 50 % verkürzt, obwohl eine um den Faktor 10 reduzierte Katalysatormenge ausreichend ist.

Nach dem erfindungsgemäßen Verfahren werden Verfahrensprodukte I erhalten, die ohne preislich und qualitativ belastende Abtrennverfahren, unmittelbar für die Verwendung als Comonomere, vor allem bei der Herstellung von Dispersionspolymerisaten, eingesetzt werden können, und die weiter vorteilhaft keine Schwermetallkomponenten von der Herstellung her enthalten. Die Verbindungen I können nach dem vorliegenden Verfahren auch als Festprodukte hergestellt werden, z.B. durch Einengen aus der Lösung.

### Durchführung der Erfindung

Für die Herstellung der Verbindungen I nach dem erfindungsgemäßen Verfahren werden Acryl- oder Methacrylsäureester der Formel II verwendet, in denen R₂ vor allem ein Alkylrest mit 1 bis 4 Kohlenstoffatomen bedeutet. Beispielsweise seien dazu Propylacrylat, n-Butylacrylat, Ethylmethacrylat, i-Propylmethacrylat, i-Butylmethacrylat, n-Butylmethacrylat und vor allem Methylmethacrylat genannt.

Als Ausgangsstoffe der Formel III kommen solche Verbindungen in Frage, in denen A oder B eine verzweigte oder unverzweigte Alkylengruppe mit 2 bis 5 Kohlenstoffatomen darstellt, z.B -(CH₂)₂-, -(CH₂)₃-, -(CH₂)₄-, -CH₂CH(CH₃)CH₂-, -CH₂C(CH₃)₂CH₂-. Die Ringgliederzahl des Heterocyclus beträgt vorzugsweise 5 und 6. Besonders vorteilhaft wird als Verbindung III 1-(2-Hydroxiethyl)-imidazolidin-2-on, das z.B. nach der US-PS 3 254 075 gut und technisch aus Aminoethylethanolamin und Harnstoff herstellbar ist, verwendet.

Der Katalysator bzw. das Katalysatorsystem KS wird zweckmäßig in Mengen, von 0,05 bis 1 Gew.-% bezogen auf die Summe der Reaktionspartner II und III angewendet. Eine hohe Selektivität an Produkt I mit R₁ = CH₃, A und B = -(CH₂)₂- wird mit 0,1 Gew.-% KS - z.B. LiOH/CaO-Gemisch - bezogen auf die Gesamtansatzmenge bei der Umesterung von Methylmethacrylat mit der dazu entsprechenden Verbindung III erreicht. Der Anteil der Komponente A am Katalysatorsystem KS (= Summe A + B) macht 5 bis 95 Gew.%, vorzugsweise 90 bis 10 Gew.-%, der Anteil der Komponente B 95 bis 5 Gew.-%, vorzugsweise 90 bis 10 Gew.% aus, speziell ist B in einem gewichtsmäßigen Überschuß gegenüber A, beispielsweise in doppelter Menge anwesend.

Vorteilhafterweise geschieht die Verwendung der Katalysatoren in feiner Verteilung z.B. in pulver- bzw. in feinkristalliner Form. Die Komponenten A und B können vor der Verwendung gemischt werden, sie können aber auch als Einzelkomponente den Reaktionsansätzen zugefügt werden.

Genannt seien z.B. Katalysatorsysteme KS gebildet aus
Lithiumoxid plus Calciumoxid
Lithiumhydroxid plus Calciumoxid
Lithiumalkoxid plus Calciumoxid
Lithiumcarbonat plus Calciumoxid
Lithiumacetat plus Calciumoxid
Lithiumfluorid plus Calciumoxid
Lithiumchlorid plus Calciumoxid
Lithiumbromid plus Calciumoxid
Lithiumjodid plus Calciumoxid
Lithiumchlorat plus Calciumoxid
Lithiummethylat plus Calciumchlorid
(wobei als Alkoxide die Methoxide, Ethoxide, tert.Butoxide besonders erwähnt seien).

Die Umsetzung von Acrylestern und/oder Methacrylestern der Formel II mit den Alkoholen der Formel III (Alkoholyse) wird bei Temperaturen zwischen 30 und 180 Grad C, vor allem zwischen 50 und 130 Grad C, in Gegenwart von 0,05 bis 1 Gew.-% KS berechnet auf das Gewicht des Reaktionsgemisches, durchgeführt.

Formal reagieren äquimolare Mengen der Reaktionspartner II und III zu den gewünschten Endprodukten I. In der Praxis hat es sich jedoch als zweckmäßig erwiesen, während der Umsetzung die Ausgangsester II stets im Überschuß zu halten. Sie werden in Mengen von 1 bis 20, vorzugsweise 2 bis 10, insbesondere 3 bis 6 Mol pro Mol III eingesetzt.

Zur Vermeidung von Polymerisationsverlusten ist es zweckmäßig, die Umsetzung und Aufarbeitung des Reaktionsgemisches in Gegenwart von Polymerisationsinhibitoren wie z.B. Phenothiazin, Hydrochinonmonomethylether und insbesondere Sauerstoff, durchzuführen.

Die Umsetzung kann unter Normaldruck, Unter- oder Überdruck ablaufen. Sie kann diskontinuierlich oder kontinuierlich erfolgen. Die Ausgangsstoffe II und III werden beispielsweise gemeinsam in Gegenwart des aus den Alkali- und/oder Erdalkaliverbindungen, insbesondere aus den Komponenten A und/oder B bestehenden Katalysatorsystems zum Sieden erhitzt und dabei kontinuierlich der abgespaltene Alkohol R₂OH, gegebenenfalls in Form seines Azeotrops mit dem Ester II abdestilliert. Je nach Reaktionstemperatur, Katalysator bzw. Katalysatormenge liegen die Umsetzungszeiten im Bereich von ca. 1 bis 7 Stunden. Es ist auch möglich, die Umsetzung in Gegenwart eines inerten Lösungsmittels, z.B. Toluol oder Cyclohexan, durchzuführen, was sich aber normalerweise erübrigt.

Nach Beendigung der Umsetzung kann überschüssiger monomerer Ester II vollkommen oder teilweise durch Abdestillieren entfernt werden.
Der dispergierte Katalysator wird üblicherweise durch Filtration, vorteilhaft vor dem Abdestillieren des meist überschüssigen monomeren Esters II, entfernt. Die Abtrennung kann aber auch erst nach teilweiser oder vollständiger Entfernung von überschüssigem monomerem Ester II erfolgen. Das in abfiltrierter Form zurückgewonnene Katalysatorsystem KS kann dann, gegebenenfalls nach Trocknung, wieder in weiteren Alkoholyseansätzen eingesetzt werden.

Ein bevorzugtes Umsetzungsprodukt ist ein solches, das aus Methylmethacrylat und 1-(2-Hydroxiethyl)-imidazolidin-2-on entsteht und so der Formel I mit R₁ = CH₃, A = -(CH₂)₂- und B = -(CH₂)₂- entspricht.

### BEISPIELE

### Beispiel 1

In einem 2 l-Vierhalsrundkolben mit aufgesetzter Kolonne und Rücklaufteiler werden 248 g 1-(2-Hydroxiethyl)imidazolidin-2-on auch als N-(Hydroxiethyl)ethylenharnstoff bezeichnet, und 1 050 g Methylmethacrylat (MMA) vorgelegt. Zur Stabilisierung setzt man 500 ppm p-Methoxyphenol und 50 ppm Phenothiazin hinzu und leitet während der Umesterung in langsamem Strom Luft durch das Reaktionsgemisch. Zunächst wird der Ansatz entwässert, indem man bei geöffnetem Rücklaufteiler das MMA-Wasser-Azeotrop so lange abzieht, bis sich eine Kopftemperatur von 99 Grad C einstellt. Dann kühlt man auf 70 - 85 Grad C ab (Sumpftemperatur) und gibt den in MMA (mengenmäßig entsprechend dem vorher abgezogenen Azeotrop) aufgeschlämmten pulverisierten Katalysator aus 0,94 g Calciumoxid und 0,36 g Lithiumhydroxid zu.

Anschließend wird bei einer Sumpftemperatur von 95 - 102 Grad C und einer Kopftemperatur von 64 - 70 Grad C die Alkoholyse durchgeführt, wobei das Methanol-MMA-Azeotrop bei einem Rücklaufverhältnis von 5 : 1 bis 5 : 2 abgenommen wird. Nach 1 - 2 Stunden ist ein Umsatz von 95 % erreicht (bestimmt über die Methanolmenge) und die Umsetzung wird abgebrochen. Der Ansatz wird abgekühlt, zur Katalysatorabtrennung filtriert und mittels Gaschromatographie (GC) analysiert:
MMA: 67 %
N-(Methacryloyloxiethyl)ethylenharnstoff: 21,6 %
N-(Hydroxiethyl)ethylenharnstoff: 4,8 %
N-(Methacryloyloxiethyl)-N'-(methacryloyl)ethylenharnstoff: 1,7 %
N-(Methacryloyloxiethyl)-N'-(2-(methoxicarbonyl)propyl)ethylenharnstoff: 0,6 %.

### Beispiel 2

Durchführung und Mengen wie in Beispiel 1. Als Katalysator wird ein Gemisch aus 0,65 g Lithiumchlorid und 0,65 g Calciumoxid eingesetzt. Die filtrierte Reaktionsmischung hat gemäß GC die folgende Zusammensetzung:
MMA: 68,2 %
N-(Methacryloyloxiethyl)ethylenharnstoff: 21,8 %
N-(Hydroxiethyl)ethylenharnstoff: 4,8 %
N-(Methacryloyloxiethyl)-N'-(methacryloyl)ethylenharnstoff: 1,7 %
N-(Methacryloyloxiethyl)-N'-(2-(methoxicarbonyl)propyl)ethylenharnstoff: 0,5 %.

### Beispiel 3

Durchführung und Mengen wie bei Beispiel 1. Als Katalysator werden 280 ppm (bez. auf Gesamteinwaage) Lithiumhydroxid verwendet. Die filtrierte Reaktionsmischung hat laut GC die folgende Zusammensetzung:
MMA: 67,7 %
N-(Meth)acryloyloxiethyl)ethylenharnstoff: 23 %
N-(Hydroxiethyl)ethylenharnstoff: 4,3 %
N-(Methacryloyloxiethyl)-N'-(methacryloyl)ethylenharnstoff: 1,6 %.
N-(Methacryloyloxiethyl)-N'-(2-(methoxicarbonyl)propyl)ethylenharnstoff: 0,6 %.

## Patentansprüche

1. Verfahren zur Herstellung von (Meth)acrylestern der Formel I 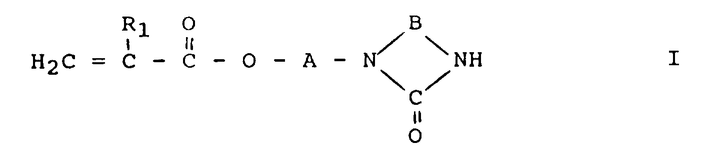 in der R₁ für Wasserstoff oder eine Methylgruppe steht und A und B unverzweigte oder verzweigte Alkylengruppen mit 2 bis 5 C-Atomen bedeuten, durch Umsetzung eines Acrylsäureesters oder Methacrylsäureesters der Formel II 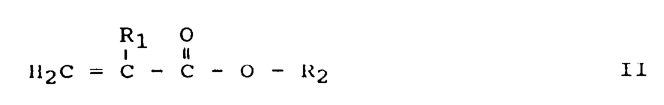 in der R₁ oben angegebene Bedeutung und R₂ die Bedeutung eines Alkylrestes mit 1 bis 4 C-Atomen haben, mit einer heterocyclischen Verbindung der Formel III 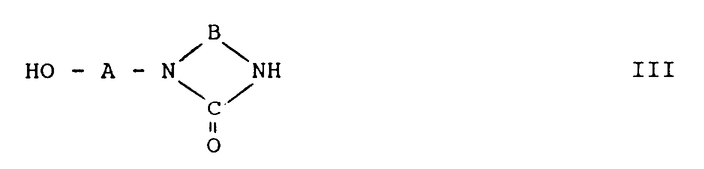 dadurch gekennzeichnet, daß die Umesterung von II mit III zu einem Acryl- oder Methacrylester der Formel I in Gegenwart von 0,05 bis 1 Gew.-% (bezogen auf die Summe von II und III) eines Katalysatorsystems KS aus Lithium- und/oder Calcium-Verbindungen durchgeführt wird.

2. Verfahren nach Anspruch 1, dadurch gekennzeichnet, daß die Umesterungsreaktion in Gegenwart eines Katalysatorensystems KS bestehend aus der Kombination der Verbindungen
A + B
wobei
A die Verbindung Liₙ Y bedeutet, worin Y für Halogenid oder Chlorat oder für Carbonat oder dem Salz einer Carbonsäure mit 1 bis 6 Kohlenstoffatomen oder für ein Alkoxid mit 1 bis 4 Kohlenstoffatomen, Hydroxid oder für Sauerstoff und n entsprechend der Wertigkeit von Y für 1 oder 2 steht und
B die Verbindung CaX_{q} bedeutet, worin X für Sauerstoff oder Chlorid und q entsprechend der Wertigkeit von X für 1 oder 2 steht,
mit der Maßgabe, daß mindestens einer der beiden anionischen Bestandteile, X und Y Sauerstoff enthalten, durchgeführt wird.

3. Verfahren nach dem Anspruch 2, dadurch gekennzeichnet, daß die Verbindung A im Katalysatorsystem KS 5 bis 95 Gew.-%, die Verbindung B 95 bis 5 Gew.-% beträgt.

4. Verfahren nach den Ansprüchen 1 bis 3, dadurch gekennzeichnet, daß das Katalysatorsystem KS aus
Calciumoxid plus Lithiumoxid, und/oder
Calciumoxid plus Lithiumhydroxid und/oder
Calciumoxid plus Lithiummethylat und/oder
Calciumoxid plus Lithium tert.butoxid und/oder
Calciumoxid plus Lithiumacetat und/oder
Calciumoxid plus Lithiumchlorid und/oder
Calciumoxid plus Lithiumbromid und/oder
Calciumoxid plus Lithiumjodid und/oder
Calciumoxid plus Lithiumchlorat und/oder
Calciumchlorid plus Lithiummethylat, besteht.

5. Verfahren nach den Ansprüchen 1 bis 4, dadurch gekennzeichnet, daß die Umsetzung von Acryl- oder Methacrylestern der Formel II mit 1-(2-Hydroxiethyl)-imidazolidin-2-on durchgeführt wird.

6. Verfahren nach den Ansprüchen 1 bis 4, dadurch gekennzeichnet, daß Methylmethacrylat als Verbindung der Formel II mit einem Alkohol der Verbindung III umgesetzt wird.

## Claims

1. A process for preparing (meth)acrylic esters of formula I 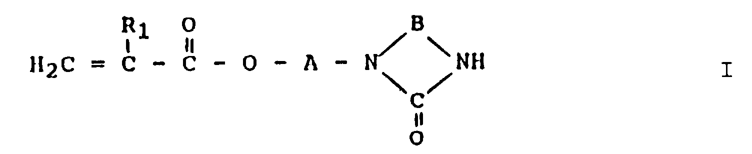 wherein R₁ is hydrogen or a methyl group and A and B are unbranched or branched alkylene groups having 2 to 5 carbon atoms, by reacting an acrylic acid ester or methacrylic acid ester of formula II 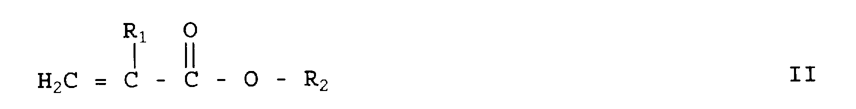 wherein R₁ has the above meaning and R₂ the meaning of an alkyl group having 1 to 4 carbon atoms, with a heterocyclic compound of formula III 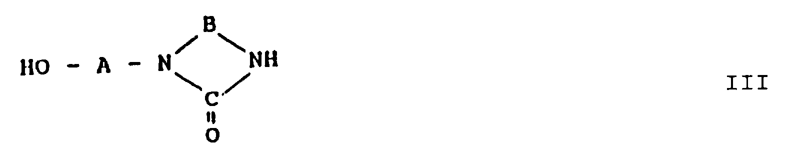 characterised in that the transesterification of II with III to form an acrylic or methacrylic ester of Formula I is carried out in the presence of 0.05 to 1 wt.% (based on the sum of II and III) of a catalyst system KS comprising lithium and/or calcium compounds.

2. A process according to claim 1, characterised in that the transesterification reaction is carried out in the presence of a catalyst system KS comprising the combined compounds
A + B
wherein
A denotes the compound Liₙ Y wherein Y is a halide or chlorate or carbonate or the salt of a carboxylic acid having 1 to 6 carbon atoms or an alkoxide having 1 to 4 carbon atoms, hydroxide or oxygen and n is 1 or 2 depending on the valence of Y and
B is the compound CaX_{q} wherein X is oxygen or chlorine and q is 1 or 2 depending on the valence of X,
with the proviso that at least one of the two anionic components X and Y contain oxygen.

3. A process according to claim 2, characterised in that the catalyst system KS comprises 5 to 95 wt.% of compound A and 95 to 5 wt.% of compound B.

4. A process according to claims 1 to 3, characterised in that the catalyst system KS comprises
Calcium oxide plus lithium oxide, and/or
Calcium oxide plus lithium hydroxide and/or
Calcium oxide plus lithium methylate and/or
Calcium oxide plus lithium tert.butoxide and/or
Calcium oxide plus lithium acetate and/or
Calcium oxide plus lithium chloride and/or
Calcium oxide plus lithium bromide and/or
Calcium oxide plus lithium iodide and/or
Calcium oxide plus lithium chlorate and/or
Calcium chloride plus lithium methylate.

5. A process according to claims 1 to 4, characterised in that the reaction of acrylic or methacrylic esters of formula II is carried out with 1-(2-hydroxyethyl)-imidazolidin-2-one.

6. A process according to claims 1 to 4, characterised in that methylmethacrylate as a compound of formula II is reacted with an alcohol of compound III.

## Revendications

1. Procédé de préparation d'esters (méth)acryliques de formule I 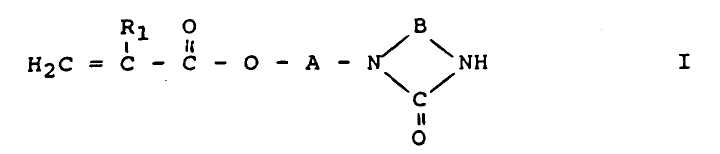 dans laquelle R₁ est mis pour un atome d'hydrogène ou un groupement méthyle et A et B représentent des groupements alkylène à 2-5 atomes de carbone non ramifiés ou ramifiés, par réaction d'un ester d'acide acrylique ou d'un ester d'acide méthacrylique de formule II 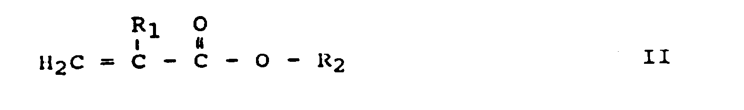 dans laquelle R₁ a la signification donnée ci-dessus et R₂ représente un reste alkyle à 1-4 atomes de carbone. avec un composé hétérocyclique de formule III 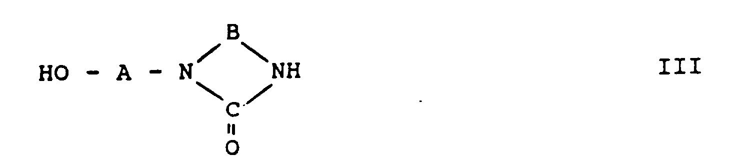 caractérisé en ce que la transestérification de II avec III pour donner un ester acrylique ou méthacrylique de formule I est effectuée en présence de 0,05 à 1% en poids (par rapport à la somme de II et III) d'un système catalytique KS à base de composés du lithium et/ou du calcium.

2. Procédé selon la revendication 1, caractérisé en ce que la réaction de transestérification est effectuée en présence d'un système catalytique KS constitué par la combinaison des composés
A + B
A représentant le composé LiₙY, où Y est mis pour un halogénure ou un chlorate, pour un carbonate ou le sel d'un acide carboxylique à 1-6 atomes de carbone, pour un alcoolate à 1-4 atomes de carbone, un hydroxyde ou pour un atome d'oxygène et n est mis pour 1 ou 2 selon la valence de Y, et
B représente le composé CaX_{q}, où X est mis pour un atome d'oxygène ou un chlorure et q est mis pour 1 ou 2 selon la valence de X,
étant spécifié que l'un au moins des deux constituants anioniques X et Y doit contenir de l'oxygène.

3. Procédé selon la revendication 2, caractérisé en ce que le composé A entre pour 5 à 95% en poids dans le système catalytique KS, le composé B y entre pour 95 à 5% en poids.

4. Procédé selon l'une quelconque des revendications 1 à 3, caractérisé en ce que le système catalytique KS se compose de:
oxyde de calcium + oxyde de lithium et/ou
oxyde de calcium + hydroxyde de lithium et/ou
oxyde de calcium + méthylate de lithium et/ou
oxyde de calcium + tert.-butylate de lithium et/ou
oxyde de calcium + acétate de lithium et/ou
oxyde de calcium + chlorure de lithium et/ou
oxyde de calcium + bromure de lithium et/ou
oxyde de calcium + iodure de lithium et/ou
oxyde de calcium + chlorate de lithium et/ou
oxyde de calcium + méthylate de lithium.

5. Procédé selon l'une quelconque des revendications 1 à 4, caractérisé en ce que l'on effectue la réaction d'esters acryliques ou méthacryliques de formule II avec de la 1-(2-hydroxyéthyl)-imidazolidine-2-one.

6. Procédé selon l'une quelconque des revendications 1 à 4, caractérisé en ce que du méthacrylate de méthyle est mis en réaction, en tant que composé de formule II, avec un alcool de formule III.
